(19) Europäisches Patentamt
European Patent Office
Office européen des brevets

(11) EP 1 561 760 B1

(12) EUROPEAN PATENT SPECIFICATION

(45) Date of publication and mention of the grant of the patent:
**26.11.2008 Bulletin 2008/48**

(51) Int Cl.:
***C08B 37/10*** *(2006.01)* ***A61K 31/727*** *(2006.01)*

(21) Application number: **05250081.6**

(22) Date of filing: **10.01.2005**

# (54) Low molecular weight heparin salt with triethanolamine

Salz von Heparin mit niedrigem Molekulargewicht mit Triethanolamin

Sel triéthanolamine d'une héparine de faible poids moléculaire

(84) Designated Contracting States:
**AT BE BG CH CY CZ DE DK EE ES FI FR GB GR HU IE IS IT LI LT LU MC NL PL PT RO SE SI SK TR**

(30) Priority: **04.02.2004 AR 0400347**

(43) Date of publication of application:
**10.08.2005 Bulletin 2005/32**

(73) Proprietor: **SYNTEX S.A.**
**Buenos Aires (AR)**

(72) Inventors:
- **Diaz, Victor Bautista**
**Luis Guillon**
**Prov. de Buenos Aires**
**1838 (AR)**
- **Martinez, Oscar Eduardo**
**Luis Guillon**
**Prov. de Buenos Aires**
**1838 (AR)**

(74) Representative: **Alcock, David**
**D Young & Co**
**120 Holborn**
**London EC1N 2DY (GB)**

(56) References cited:
**EP-A2- 0 268 885**
**DE-A1- 3 020 220**
**US-A- 4 966 894**
**US-A- 5 013 724**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention refers to a new chemical substance obtained by salification of depolymerized acid polysaccharides, particularly, low molecular weight heparin with the organic base TRIETHANOLAMINE. Moreover, pharmaceutical formulations containing said low molecular weight heparin salt (LMWH) with triethanolamine (TEA) are described for its therapeutic delivery by local route. Moreover, the present invention refers to new process for preparing said low molecular weight heparin salt with triethanolamine. A process for eliminating hygroscopicity of heparin salt, pharmaceutical compositions for local use in antithrombotic therapy and uses therein are also described.

The acid polysaccharides, in general, and particularly heparin, may be depolymerized by well reported processes in US-A- 4977250/EP-A-0268885.

Said documents describe a process for the chemical depolymerization of polysaccharides and, particularly, glycosaminoglycan sulfates, comprising heparin, heparan sulfate and dermatan sulfate, as well as the use of the products which are obtained as antithrombotic agents.

Although the depolymerization reaction must be carried out under highly specific conditions, the process is reproducible in practice at technical scale, allowing the obtainment of depolymerized compounds of doubtless value as antithrombotic agents and well recognized by medical practice (particularly, low molecular weight heparin).

The documents mentioned allow the obtainment, starting from injectable heparin USP grade, of low molecular weight heparin in accordance with the European Pharmacopoeia specifications for its use as injectable antithrombotic agent.

Other processes for the chemical depolymerization of acid polysaccharides, particularly glycosaminoglycan sulfates, are detailed in AR-A-243540, ES-A-2007373, IT-A-1224260, BE-A-8700861 and permit, likewise, to obtain low molecular weight heparin starting from injectable heparin USP/EP grade as raw material.

In any case, the depolymerized products obtained by the above methods are sodium salts of low molecular weight heparin, which is not capable of being dermically delivered as it is a substance of strong ionic nature, of significant molecular weight (in average 4000 - 6000 Da), low affinity to fats and lipids in general and with a no partitioning into water-octanol.

These strongly ionic characteristics of low molecular heparin (and even more, of the starting heparin, due to its higher molecular weight) have severely limited the use of this substance as local delivery antithrombotic agent and, in fact, to date there are no pharmaceutical preparations which are known to contain low molecular weight heparin for local use, for the alleviation or prevention of varicose veins, hemorrhoids, traumatic or post-surgical hematomas (plastic surgery or facial reconstruction surgery).

The present invention describes in detail a new compound, low molecular weight heparin salt with triethanolamine, as well as alternative processes for its preparation, wherein triethanolamine is used in order to increase the lipophilicity of the subject acid polysaccharide, allowing its passage through the skin, by the formation of a stable salt with the polysaccharide, replacing an inorganic counterion which always salifies the heparin and which is preferably selected from sodium, potassium, calcium, magnesium and ammonium, thereby considerably reducing its hydrophilicty and increasing its lipophilicity. The inorganic counterion most preferably used is sodium.

It is inferred therefrom that the low molecular weight heparin structure itself, is not altered by the salification process with triethanolamine (TEA) and, therefore, it does not suffer any variation on its measurable biological properties (anti Xa, anti IIa), as not being the corresponding stoichiometric variation.

For research purposes, the low molecular weight heparin salts were prepared and tested, particularly the diethylamine, trietylamine, tributylamine, monoethanolamine, diethanolamine and choline salts.

In all cases, triethanolamine (TEA) was the chosen organic base for the carrying out of the invention, due to its recognized use in pharmaceutical and cosmetic dermal preparations (as well as in hair care products) for producing no dermal irritation and for greatly allowing skin penetration of the acid polysaccharide salified with said triethanolamine.

The salt obtained from the reaction of the triethanolamine (TEA) with the low molecular weight heparin (LMWH) is an amber colored substance, with a waxy appearance, extremely difficult to manipulate due to its high hygroscopicity. These characteristics of the LMWH -TEA salt make extremely difficult its precise dosage in the preparation of the pharmaceutical end products (creams, gels, ointments).

Therefore, another characteristic of the present invention is the lyophilization of said LMWH -TEA salt (as it is obtained from its obtainment processes, in aqueous solution) in the presence of Mannitol or Sorbitol. Both monosaccharide polyalcohols are chemically indifferent to the said LMWH -TEA salt as they do not contain aldehyde groups (potential or not) which may react with the amine TEA providing substances like SCHIFF bases, or other type of parasitic reaction, they do not present dermal irritation, they are biologically compatible and chemically stable, as well as commercially available.

Particularly, it was experimentally found that in the end solid lyophilizate, a very adequate ratio of LMWH -TEA / Mannitol is 60:40 weight by weight, which offers a non-hygroscopic lyophilized solid, well manipulated, stable and susceptible of being well dosed by weighing, in the preparation of pharmaceutical products.

Therefore, the detailed characteristic of the present invention includes concepts and methods for reducing the hygroscopicity of the LMWH -TEA salt by lyophilization on a inert carrier (preferably Mannitol or Sorbitol) chemically indifferent and biologically compatible.

The drawings which illustrate the present invention represent:

Figure 1 shows a NMR spectrum (13C) of the LMWH - TEA.
Figure 2 shows the tail of a sample rat following 24 hours of the application of an injection of k carrageenan.
Figure 3 represents the tail of a rat following 24 hours of the application of an injection of k carrageenan treated with 0, 5 g of the cream I which is defined below.
Figure 4 represents the tail of a rat following 24 hours of the application of an injection of k carrageenan treated with 0, 5 g of the cream II which is defined below.
Figure 5 represents the tail of a rat following 24 hours of the application of an injection of k carrageenan treated with 0, 5 g of the cream III which is defined below.

The following examples, which are not limited by the generality of the application, describe processes and products obtained.

## Example 1

Depolymerization of Heparin

100 g of injectable heparin USP/EP grade are dissolved in distilled water, in a sufficient amount to obtain 500 ml of 20% solution.

The solution is placed in a four entrance balloon provided with stirring, pH-meter electrode, heating and reflux refrigerant. The aqueous solution was warmed at 94 - 96°C and then a 1 M hypochlorous acid solution is added at a temperature of 0/5°C (prepared by fitting to pH = 6 with HCl of 80g/l sodium hypochlorite solution previously refrigerated). 290 ml of 1M solution of HClO is added in a 60 minute reaction, while keeping the pH constant from 5.0 to 6.0 adding HCl or NaOH in aqueous solution, as appropriate.

After the 60 minute reaction has been completed, 3% sodium chloride is added and the reaction product precipitates quickly adding 2 volumes of ethanol under vigorous stirring, thereby producing the immediate blockade of the depolymerization reaction.

Salification with TEA

Once the hydroalcoholic supernatant is decanted, the precipitated polysaccharide (LMWH sodium salt) is dissolved in distilled water in a sufficient amount to provide a 5% w/v solution. The pH is regulated to 5.0-5.5 with acetic acid solution and the solution is subjected to diafiltration (cut off 5000 Da) versus 5% TEA solution in distilled water (pH adjusted to 5.0 - 5.5 with acetic acid). 20 vol of triethanol ammonium acetate are thereby used.

Subsequently, the diafiltered solution is subjected to diafiltration with 10 volumes of distilled water to remove the free TEA residues from the solution.

The solution is discharged from the ultrafilter and lyophilized, providing 140 g waxy, light amber, highly hygroscopic product, that is the heparin salt of low molecular weight with triethanolamine, which presents the following comparative analysis:

| | *Injectable Heparin* | *LMWH-TEA* |
|---|---|---|
| EP Strength | 182 iu/mg | --- |
| anti Xa Strength | --- | 51 iu/mg |
| Anti IIa strength | --- | 24 iu/mg |
| Sulfur (S) | 11.7% | 6.8% |
| S/$COO^-$ Ration | 2.1 | 2.1 |
| Triethanolamine | --- | 49 % |
| Sodium (Na) | 11.0% | 19 ppm |
| pM (HPSEC) | 13500 Da | 5400 Da |
| < 8000 Da | --- | 82 % |
| 3000 - 8000 Da | --- | 53 % |
| < 3000 Da | --- | 25 % |

Note: The anti Xa and anti IIa biological strengths were measured according to the European Pharmacopoeia EP2002; "Low Molecular Mass Heparin", as well as the mean molecular weights (weighed average Mw) and the molecular weight distribution. Moreover, the standards and gages are those strictly cited in the mentioned literature. It is noted that in the run buffer for the chromatographic test by HPLC ($Na_2SO_4$, 0.2M pH 5.0) the TEA is replaced by $Na^+$ whereby the declared value is that of the sodium polysaccharide fraction and not the one of the LMWH-TEA salt.

**Example 2**

Depolymerization of Heparin

100 g of injectable heparin USP/EP grade are dissolved in distilled water, in a sufficient amount to provide 500 ml of 20% w/v solution and it is transferred to a balloon provided with heating, stirring and refrigerant at reflux.

A reagent comprised of 20 ml of 30% $H_2O_2$ and 1 ml of aqueous solution of 1.5% Fe $SO_4.7H_2O$ is warmed at 80°C and is added under stirring.

Heating and stirring are continued for a 40 minute period, 2% NaCl is added and the reaction is immediately stopped precipitating the depolymerized polysaccharide by addition of 2 vol. of ethanol.

The obtained precipitate is dissolved in distilled water in a sufficient amount to provide a 10% solution and this solution is introduced in a chromatografic column of 1L capacity (∅ 5.2 cm x 50 cm height) containing ionic exchange resin Amberlite® IR-120 (Rohm Haas) (phase $H^+$) , at a flux of 0.42 $cm^3$/ $cm^2$ x minute. The acid eluant is collected onto an ice bath vessel with pH adjustment to 5.0 - 5.5 value adding dropwise triethanolamine NF grade. Once the passage is completed, the column is washed with distilled water until neutral eluant is obtained. Washings and eluants were collected and pH was adjusted to pH 5.0 - 5.5 by the addition of triethanolamine.

It is lyophilized, obtaining 156 g of the heparin salt of low molecular weight with triethanolamine, LMWH -TEA salt, highly hygroscopic, waxy and amber.

The following is a comparative analysis:

| | ***Injectable Heparin*** | ***LMWH-TEA*** |
|---|---|---|
| EP Strength | 180 iu/mg | --- |
| anti Xa Strength | --- | 50 iu/mg |
| anti IIa Strength | --- | 24 iu/mg |
| Sulfur | 11.9 % | 6.8 % |
| S/$COO^-$ Ratio | 2.1 | 2.1 |
| Triethanolamine | --- | 50 % |
| Sodium | 12.0% | 280 ppm |
| Mw (HPSEC) | 12000 Da | 4800 Da |
| < 8000 Da | --- | 81 % |
| 3000 - 8000 Da | --- | 46 % |
| < 3000 Da | --- | 35 % |

The same explanations of Example N° 1 apply to this example.

**Example 3**

Lyophilization in the presence of Mannitol: solving the problem of hygroscopicity inherent to LMWH-TEA.

18.6 g of LMWH-TEA obtained in Example N°2 are dissolved in distilled water in a sufficient amount to provide 125 ml of 15% w/v solution. 12.40 g of mannitol USP are added under stirring, which is very well dissolved at room temperature, if the initial solution does not have a concentration higher than 15%, as in this case.

The solution is lyophilized to provide a crystalline dried powder, that can be subjected to grinding, of highly reduced hygroscopicity. The milled powder is flowable and does not become clotted.

The following Table of analysis is given for comparative purposes:

| | ***LMWH-TEA*** | ***LMWH -TEA / MANNITOL*** |
|---|---|---|
| anti Xa Strength | 50 iu/mg | 30 iu/mg |
| anti IIa Strength | 24 iu/mg | 14 iu/mg |

(continued)

| | LMWH-TEA | LMWH -TEA / MANNITOL |
|---|---|---|
| Sulfur | 6.8 % | 4.2 % |
| $S/COO^-$ Ratio | 2.1 | 2.1 |
| Triethanolamine | 50.0 % | 30.0 % |
| Sodium | 280 ppm | 170 ppm |
| Mw (HPSEC) | 4800 Da | 4800 Da |
| < 8000 Da | 81 % | 81 % |
| 3000 - 8000 Da | 46 % | 46 % |
| < 3000 Da | 35 % | 35 % |
| Mannitol | ----- | 40 % |

## Example 4

Depolymerization of Fractions of Low Strength Heparin

In preparing injectable heparin, byproducts are obtained containing heparins of low anticoagulant activity together with dermatan sulfate and chondroitin sulfate at varying concentrations. The depolymerized products of these byproducts may be salified with triethanolamine and used as antithrombotics locally.

This example, is not a limitation of the generality of the cases, starts from a fraction of low strength heparin, characterized by the following analysis (0213409IIA)

| | |
|---|---|
| USP Strength: | 45 iu/mg |
| Specific Optic Rotation: | + 41° |
| Composition (Electrophoresis): | 12% "Slow moving" (SM) Fraction 77% "Fast moving" (FM) Fraction 11 % Dermatan Sulfate |

The electrophoretic run was carried out according to Cappelletti, R et al, Anal. Biochem. 99 311-315 (1979).

50 g of the above fraction are dissolved in distilled water in a sufficient amount to provide 250 ml of 20% solution. The solution is placed into a 500 ml capacity balloon equipped with thermometer, heating, stirring and refrigerant at reflux. It is warmed to 95 - 98°C and then 580 ml of hypochlorous acid solution 1 M are added (obtained by pH adjustment to pH 6 of 80 g/l NaClO solution with hydrochloric acid at a temperature from 0 - 5°C) for 60 minutes, while keeping pH constant at all times from 5.0 - 6.0 values adding a hydrochloric acid or sodium hydroxide solution, as appropriate. Once the addition of the solution is completed, it is precipitated adding two volumes of ethanol. The depolymerized polysaccharide which constitutes the precipitate is dissolved in distilled water in a sufficient amount to provide a 5% solution and pH is adjusted to 5.0 - 5.5 value with acetic acid solution. The clear solution thus obtained is subjected to a diafiltration process in a appropriate ultrafilter, equipped with a cassette of UF cut off 1000 Da, versus 5% solution of triethanolamine in water, of pH adjusted to 5.0 - 5.5 with acetic acid.

20 volumes of dialyzer solution are thereby passed and once this step is completed, the diafiltration is continued with the passage of 10 vols. of distilled water to remove free TEA from the concentrate retained by the UF membrane. The solution is discharged from the UF and lyophilized providing 42 g of a product of waxy consistency and amber colored, highly hygroscopic, which is the triethanolamine salt of the initial depolymerized heparinic fraction.

The product presents the following comparative analysis.

| | |
|---|---|
| Anti Xa strength | 73 iu/mg (EP Method) |
| Anti IIa strength | 13 iu/mg (EP Method) |
| Sulfur | 4.8% |
| $S/COO^-$ Ratio | 1.7 |
| Electrophoresis | 90% FM Fraction |
| 10% Dermatan sulfate | |
| Triethanolamine | 49.0% |
| MW (HPSEC) | 5060 Da |
| <8000 Da | 82.0% |
| <3000 Da | 30.0% |

(continued)

| | |
|---|---|
| <2000 Da | 14.0% |
| Specific Optic Rotation | + 20° |

**Characterization of the LMWH-TEA substance**

1) Theoretic Content of TEA in the LMWH-TEA salt

Heparin is defined as a family of polysaccharides, which chains are formed alternating residues of uronic acid and D-glucosamine bonded one to the other by 1-4 bonds and with several grades of sulfating. The uronic acid residue is L-iduronic or D-glucuronic and the glucosaminic residue may be N-sulfated or N-acetilated.

(Casu, B; "Methods of Structural Analysis" in "Heparin, Chemical and Biological Properties, Clinical Applications", Lane, D; Lindhal, U., Eds; E. Arnold (1989) 25 - 49)

Therefore, only one approximate formula can be obtained for the disaccharide unit (which does not really exist, due to the molecular microheterogenicity of the heparin). The following formula is in good agreement with the experimental analytical data of an injectable heparin which meets the USP - EP specifications:

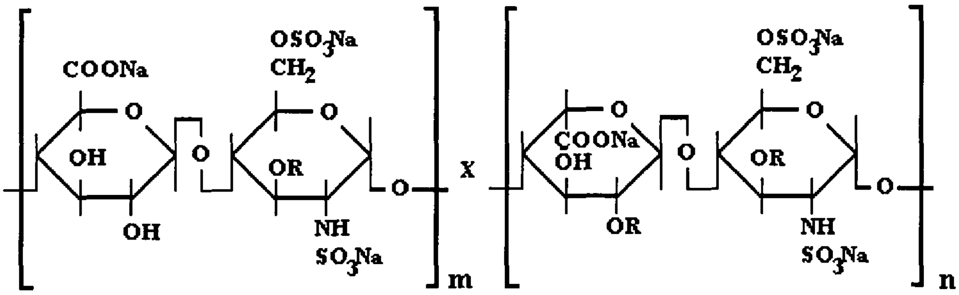

wherein: R = $NaSO_3$ or H
m = 1 and n ≥ 4
m + n = approx. 9

- Molecular formula of the unit disaccharide (for R = H)

$$C_{12}H_{15}O_{16}NS_2Na_3$$

- Molecular weight of the unit disaccharide (for R = H): 562

The depolymerization reaction, which enables obtaining low molecular weight heparin starting from injectable heparin, does not introduce marked modifications throughout the polysaccharide chain, except for the molecular weight reduction and the properties related thereto (for instance, viscosity).

Therefore, in the present theoretical calculation of the TEA content in LMWH - TEA, we will accept that the unit disaccharide remains intact with the sole change of $Na^+$ for the ion Triethanolammonium.

Thus, 100 g of LMWH sodium salt contain, according to the above scheme, 12,1 g $Na^+$ (0.527 gram atom of $Na^+$) which shall be replaced, in the composition of the triethanolamine salt, by 0.52 mol triethanolamine (under the form of 0.52 mol of the ion triethanolammonium).

The molecular weight of the TRIETHANOLAMMONIUM ion is 150.2 therefore 0.527 mol triethanolammonium = 150.2 x 0.527= 79.15 g

In the ionic exchange:

100 g LMWH Na lose 12.1 g $Na^+$ and gain 79.15 g of triethanolammonium, producing 167.0 g of LMWH -TEA.

Therefore, the theoretical content of $TEA = \frac{79.15 \, x \, 100}{167} = 47.40 \%$

2) <u>Theoretical Content of S in LMWH -TEA</u>

According to the unit disaccharide accepted as starting point, the % S = 11.4%, in good accordance with the analytical data that presents an injectable heparin which meets the USP-EP specifications.

In the depolymerization of the injectable heparin and in the subsequent salification of the depolymerized product, with TEA, S is not lost, so the theoretical data of S in LMWH-TEA should be:

$$\frac{11.4 \, x \, 100}{167} = 6.8 \% \text{ theoretical S.}$$

3) <u>Theoretical Calculation of the biological strengths in LMWH -TEA</u>

Low molecular weight heparins (sodium salt) obtained by radical fragmentation methods of injectable heparin, such as those cited in European Pat. 268885, US PAT 4977250, present an anti Xa strength ≅ 90 iu/mg and an anti IIa strength ≅ 40 iu/mg.

Taking into account the dilution which undergo these values because of the molecular weight increase of the unit disaccharide when eliminating $Na^+$ and salifying with triethanolamine, we will have that:

100 g LMWH-Na = 167 g LMWH-TEA

$$\text{therefore, theoretical anti Xa strength} = \frac{90}{1.67} = 54 \text{ iu.}/mg$$

$$\text{theoretical anti IIa strength} = \frac{40}{1.67} = 24 \text{ iu}/mg$$

4) <u>Comparison of theoretical analytical data and formulae of the unit disaccharides</u>

| | *INJECTABLE HEPARIN* | *LMWH-Na* | *LMWH-TEA* |
|---|---|---|---|
| ***Sulfur*** | 11.4% | 11.4% | 6.8% |
| ***S/COO⁻*** | 2.1 | 2.1 | 2.1 |
| ***Na⁺*** | 12.1% | 12.1% | --- |
| ***Triethanolamine*** | --- | --- | 47.4% |
| ***MW (HPSEC)*** | 12000 - 13500 | 4000 - 6000 | 4000 - 6000 |

(continued)

| | *INJECTABLE HEPARIN* | *LMWH-Na* | *LMWH-TEA* |
|---|---|---|---|
| ***Molecular Formula of the disaccharide residue*** | $C_{12}H_{15}O_{16}NS_2Na_3$ | $C_{12}H_{15}O_{16}NS_2Na_3$ | $C_{12}H_{15}O_{16}NS_2[NH(CH_2CH_2OH)_3]$ |
| ***Molecular weight of the disaccharide residue*** | 563 | 563 | 944 |

5) Comparison of theoretical and experimental data in LMWH-TEA

| | *Theoretical* | *Experimental* |
|---|---|---|
| ***Triethanolamine*** | 47.4% | 48 - 50% |
| ***Sulfur*** | 6.8% | 6.8% |
| ***S/ COO*** | 2.1 | 2.1 |
| ***anti Xa*** | 54 iu/mg | 50 - 51 iu/mg |
| ***anti IIa*** | 24 iu/mg | 24 iu/mg |

6) Spectroscopic characterization (13C-NMR) of LMWH -TEA

The sample of low molecular weight heparin, triethanolamine salt, was dissolved in $H_2O$ : $D_2O$ : 1: 1 at conc. of 180 mg/ml. The spectrum of nuclear magnetic resonance (13C) was recorded on a spectrometer (13C) BRUCKER AM-500 (13C - 125 MHz), as illustrated in Figure 1.

Signals of triethanolamine are observed at 56.1 and 56.3 ppm, subsequently determining the relative concentration of iduronic acid 2 sulfate with regard to total uronic acid based upon relative integration of signals of C1 to 100.1 and 102.6 ppm (sulfated and free respectively). In the same manner, total glucosamine N -sulfate / glucosamine (59; 54.6 y 53 ppm) was determined. Data obtained are plotted as follows:

| | *Idu 2OS* | *Glu NS* | *Glu 6OS* | *Glu N/ Idu H* |
|---|---|---|---|---|
| **LMWH-TEA** | 75% | 85% | 77% | 1:1,67 |

7) Analytical Techniques Used

- The anticoagulant strength of injectable heparin was determined according to European Pharmacopoeia 2002 (EP strength) and to USP 26 (USP strength)
- Anti Xa and anti IIa strengths were determined according to the literature of low molecular weight heparins (European Pharmacopoeia 2002)
- Distribution of molecular weights and mean ponderal molecular weight were determined according to the literature of low molecular weight heparins (European Pharmacopoeia 2002), therefore, in the run buffer, TEA is replaced by $Na^+$ and the data obtained then pertain to polysaccharide sodium salt and not to TEA salt.
- Triethanolamine is dosed by potentiometric titration.

8. -Experimental Pharmacology; efficiency test.

Compound LMWH-TEA according to the present invention was subjected to pharmacological tests to verify its efficacy.

With such criteria, Wister rats (female) raised in the laboratory and sanitarily controlled were used as test mammal.

Particularly, the thrombosis model developed consists of generating an experimental thrombosis in the tail of the test rat by intravenous injection (right caudal vein) of a thrombogenic substance such as κ - carrageenan. Without treatment, the tail of the animal totally forms thrombus without remission over time and ends up with necrosis after 3 - 4 days.

However, local treatment with a cream of local use containing LMWH-TEA at the rate of 1000 u antiXa/gram of cream shows a highly significant restitutive activity much higher than the one demonstrated by local heparin and LMWH sodium salt when administrated under same conditions.

Particularly, this test was carried out according to the general outlines in Beckmeier et al, Agents and Actions, 16, 446 (1985), as follows:

a. Animals: female rats of 200 - 300 grams weight.

b. Thrombosing Solution: κ carrageenan Type III solution (Sigma C 1263) 1 mg / ml in sterile physiologic solution. 1 ml / kg of animal (right caudal vein of the tail) is injected (0.25 ml).

c. Thrombus induction: a tourniquet is placed near the tail base (15 cm from the end thereof) which was previously shaved. After 1 minute a thrombosing solution is injected and the animal tail is immediately immersed for 30 seconds in ice water, water is removed, it is dried and the tourniquet is kept for an additional 30 seconds. Then, the animal is kept for 2 minutes in a chamber at 10° C.

d. Efficiency test: Then, an experimental cream (0.5 g) containing 1000 units anti Xa /gram of LMWH TEA, sodium LMWH and 1000 IU / gram of local heparin respectively, is administered along the tail that has suffered an infarct to each group of animals (three per cream preparation). Another three animals do not receive treatment and are considered controls.

The animals were controlled 2, 4, 18, 24 and 72 hours post-injection of κ-carrageenan measuring in each case the portion of tail that has suffered an infarct in millimeters (mm).

The results which were obtained were the following:

| **Time** | **2 h** | **4 h** | **18 h** | **24 h** | **72 h** |
|---|---|---|---|---|---|
| Control | 134 | 134 | 134 | 134 | 133 |
| **I** | 18 | 13 | (*) | ----- | ----- |
| **II** | 44 | 44 | 43 | 40 | 39 |
| **III** | 110 | 100 | 92 | 92 | 86 |
| Cream I: LMWH -TEA 1000 IU anti Xa /gram<br>Cream II: LMWH Na 1000 IU anti Xa /gram<br>Cream III: Local Heparin 1000 IU /gram<br>(*) very slight stain | | | | | |

For clarity reasons, Figures 2 to 5 are attached which are taken 24 h post-injection in each treated group and controls.

In this test, creams for local use were prepared according to the following formula, which is not limiting of the method:

| | ***Cream I*** | ***Cream II*** | ***Cream III*** |
|---|---|---|---|
| Glyceryl monostearate | 100 g | 100 g | 100 g |
| Dodecyl oleate | 60 g | 60 g | 60 g |
| Ketostearyl alcohol | 60 g | 60 g | 60 g |
| Ketomacrogol | 30 g | 30 g | 30 g |
| LMWH -TEA | $10^6$ anti Xa | ---- | ---- |
| LMWH Na | ---- | $10^6$ anti Xa | ---- |
| Local Heparin | ---- | ---- | $10^6$ IU |
| Nipagin | 1.6 g | 1.6 g | 1.6 g |

(continued)

| | *Cream I* | *Cream II* | *Cream III* |
|---|---|---|---|
| Nipasol | 0.4 g | 0.4 g | 0.4 g |
| Sterile distilled water q.s. | 1000 g | 1000 g | 1000 g |

The test enables the documentation of the marked efficacy of the dermal preparation identified as *Cream I* (LMWH -TEA), over the other two creams, with the total abatement of the symtoms of thrombosis being observed after 18 hours.

**Claims**

**1.** A heparin salt of low molecular weight with triethanolamine useful as therapeutic-antithrombotic agent for local administration, wherein at least 60% of the total mass of the heparin salt has a molecular weight of less than 8000 Da and an average molecular weight from 4000 to 6000 Da, said salt also having a content of organic sulfur from 6.1 to 7.5 weight % (theoretical 6.8 weight %) and a triethanolamine content from 42.6 to 52.1 weight % (theoretical 47.4 weight %).

**2.** A process for preparing a heparin salt according to Claim 1, wherein the process comprises removal by diafiltration of the inorganic counterion of low molecular weight heparin with aqueous solution from 4 to 6% of triethanolamine at pH from 5 to 5.5.

**3.** A process according to Claim 2, wherein the low molecular weight heparin is obtained by thermolytic decomposition of the hypochlorous acid in 1M aqueous solution at a temperature from 0 to 5°C on the 15- 25% aqueous solution of injectable heparin, at pH from 4 to 7 and a temperature from 70 to 100°C at reflux.

**4.** A process for preparing a heparin salt according to Claim 1, wherein the process comprises removing the inorganic counterion of the low molecular weight heparin treating with ion exchange resin in acid phase and subsequent neutralization of the acid polysaccharide eluted with base triethanolamine (99%) adjusting pH of the eluate from 5.0 to 5.5 at a temperature from 15 to 25°C.

**5.** A process according to Claim 2, 3 or 4, wherein the inorganic counterion is selected from sodium, potassium, calcium, magnesium and ammonium.

**6.** A process according to Claim 4, wherein the low molecular weight heparin is obtained by thermolytic decomposition of an aqueous solution of hypochlorous acid at a temperature from 0 to 5°C on an aqueous solution of 15 - 25% concentration of injectable heparin at pH from 4 to 7 and a temperature from 70 to 100°C at reflux.

**7.** A process according to Claim 2 or 4, wherein the low molecular weight heparin is obtained through the radical decomposition of hydrogen peroxide catalized by ferrous ions on the injectable heparin, being the concentration of ferrous ion from 10 to 100 ppm, the concentration of hydrogen peroxide in a molar ratio of $H_2O_2$/polysaccharide from 6-15, pH from 4 to 7 and a reaction temperature from 60 to 120°C.

**8.** A process for eliminating hygroscopicity of a low molecular weight heparin salt with triethanolamine according to Claim 1, wherein the process comprises dissolving said salt in water until reaching a concentration of 13 to 17%, adding mannitol or sorbitol in an amount equivalent to 30 - 50 parts each 60 parts of said salt, dissolving all in aqueous solution and lyophilize.

**9.** A pharmaceutical composition for local use in antithrombotic therapy which comprise the low molecular weight heparin salt with triethanolamine according to Claim 1, wherein said salt is present in pharmaceutically active amounts from 100 to 2000 international units Anti $X_A$/g of composition of local use diluted in an adequate excipient.

**10.** A pharmaceutical composition according to Claim 9, wherein the excipient is selected from aqueous gels of carbopolymers, creams or emulsions of the oil-in-water type, or ointments of aqueous or hydroalcoholic base.

**11.** Use of low molecular weight heparin salt with triethanolamine for the manufacture of a medicament as antithrombotic therapeutic agent of local administration, wherein that it is used for the treatment, alleviation or prevention of varicose veins, hemorrhoids, traumatic or post-surgical hematomas or phlebitis.

**12.** Use according to Claim 11, wherein the post-surgical hematoma is derived from a plastic surgery or facial reconstruction.

**13.** Use according to Claim 11, **characterized in that** the phlebitis is caused by the intravenous infusion of chemotherapeutic agents.

## Patentansprüche

**1.** Heparinsalz mit niedrigem Molekulargewicht mit Triethanolamin, welches als therapeutisches antithrombotisches Mittel für die lokale Verabreichung geeignet ist, wobei wenigstens 60% der Gesamtmenge des Heparinsalzes ein Molekulargewicht von weniger als 8000 Da und ein mittleres Molekulargewicht von 4000 bis 6000 Da haben, wobei das Salz auch einen Gehalt an organischem Schwefel von 6,1 bis 7,5 Gewichts-% (theoretisch 6,8 Gewichts-%) und einen Triethanolamingehalt von 42,6 bis 52,1 Gewichts-% (theoretisch 47,4 Gewichts-%) hat.

**2.** Verfahren zum Herstellen eines Heparinsalzes nach Anspruch 1, wobei das Verfahren das Entfernen des anorganischen Gegenions von Heparin mit niedrigem Molekulargewicht mittels Diafiltration mit einer wäßrigen Lösung von 4 bis 6% Triethanolamin bei einem pH-Wert von 5 bis 5,5 umfaßt.

**3.** Verfahren nach Anspruch 2, wobei das Heparin mit niedrigem Molekulargewicht durch thermolytischen Abbau der hypochlorigen Säure in 1 M wäßriger Lösung bei einer Temperatur von 0 bis 5°C mit der 15- bis 25%-igen wäßrigen Lösung von injizierbarem Heparin bei einem pH-Wert von 4 bis 7 und einer Temperatur von 70 bis 100°C unter Rückfluß erhalten wird.

**4.** Verfahren zum Herstellen eines Heparinsalzes nach Anspruch 1, wobei das Verfahren das Entfernen des anorganischen Gegenions des Heparins mit niedrigem Molekulargewicht durch Behandeln mit Ionenaustauscherharz in Säurephase und anschließendes Neutralisieren des sauren Polysaccharids, eluiert mit Triethanolaminbase (99%), zum Einstellen des pH-Werts des Eluats auf 5,0 bis 5,5 bei einer Temperatur von 15 bis 25°C umfaßt.

**5.** Verfahren nach Anspruch 2, 3 oder 4, wobei das anorganische Gegenion unter Natrium, Kalium, Calcium, Magnesium und Ammonium ausgewählt ist.

**6.** Verfahren nach Anspruch 4, wobei das Heparin mit niedrigem Molekulargewicht durch thermolytischen Abbau einer wäßrigen Lösung von hypochloriger Säure bei einer Temperatur von 0 bis 5°C mit einer wäßrigen Lösung mit einer Konzentration von 15-25% an injizierbarem Heparin bei einem pH-Wert von 4 bis 7 und einer Temperatur von 70 bis 100°C unter Rückfluß erhalten wird.

**7.** Verfahren nach Anspruch 2 oder 4, wobei das Heparin mit niedrigem Molekulargewicht durch den radikalischen Abbau von Wasserstoffperoxid, katalysiert durch Eisen(II)-Ionen, auf dem injizierbaren Heparin erhalten wird, wobei die Konzentration an Eisen(II)-Ionen 10 bis 100 ppm beträgt. die Konzentration von Wasserstoffperoxid in einem molaren Verhältnis von $H_2O_2$/Polysaccharid 6-15 beträgt, der pH-Wert 4 bis 7 beträgt und eine Reaktionstemperatur 60 bis 120°C beträgt.

**8.** Verfahren zum Eliminieren von Hygroskopie eines Heparinsalzes mit niedrigem Molekulargewicht mit Triethanolamin nach Anspruch 1, wobei das Verfahren das Lösen des Salzes in Wasser bis zum Erreichen einer Konzentration von 13 bis 17%, das Zugeben von Mannitol oder Sorbitol in einer Menge von 30-50 Teilen pro 60 Teilen des Salzes, das Lösen des Ganzen in einer wäßrigen Lösung und Lyophilisieren umfaßt.

**9.** Pharmazeutische Zusammensetzung für die lokale Anwendung bei der Antithrombosetherapie, welche das Heparinsalz mit niedrigem Molekulargewicht mit Triethanolamin nach Anspruch 1 umfaßt, wobei das Salz in pharmazeutisch aktiven Mengen von 100 bis 2000 internationalen Einheiten Anti-$X_A$/g Zusammensetzung zur lokalen Verwendung, verdünnt in einem geeigneten Hilfsstoff, vorliegt.

**10.** Pharmazeutische Zusammensetzung nach Anspruch 9, wobei der Hilfsstoff unter wäßrigen Gelen von Carbopolymeren, Cremes oder Lösungen des Öl-in-Wasser-Typs oder Salben mit wäßriger oder alkoholisch-wäßriger Basis ausgewählt ist.

**11.** Verwendung eines Heparinsalzes mit niedrigem Molekulargewicht mit Triethanolamin zur Herstellung eines Medikaments als antithrombotisches therapeutisches Mittel für die lokale Verabreichung, welches zur Behandlung, Linderung oder Prävention von Krampfadern, Hämorrhoiden, traumatischen oder postoperativen Hämatomen oder Phlebitis verwendet wird.

**12.** Verwendung nach Anspruch 11, wobei das postoperative Hämatom durch plastische Chirurgie oder Gesichtsrekonstruktion entstanden ist.

**13.** Verwendung nach Anspruch 11, **dadurch gekennzeichnet, daß** die Phlebitis durch die intravenöse Infusion von chemotherapeutischen Mitteln verursacht wird.

## Revendications

**1.** Sel d'héparine de bas poids moléculaire avec la triéthanolamine, utile comme agent thérapeutique antithrombotique pour administration locale, dans lequel au moins 60 % de la masse totale du sel d'héparine a un poids moléculaire inférieur à 8000 Da et un poids moléculaire moyen de 4000 à 6000 Da, ledit sel ayant également une teneur en soufre organique de 6,1 à 7,5 % en poids (valeur théorique de 6,8 % en poids) et une teneur en triéthanolamine de 42,6 à 52,1 % en poids (valeur théorique de 47,4 % en poids).

**2.** Procédé pour préparer un sel d'héparine selon la revendication 1, dans lequel le procédé comprend l'élimination par diafiltration du contre-ion inorganique d'héparine de bas poids moléculaire avec une solution aqueuse de 4 à 6 % de triéthanolamine à pH 5 à 5,5.

**3.** Procédé selon la revendication 2, dans lequel l'héparine de bas poids moléculaire est obtenue par décomposition thermolytique de l'acide hypochloreux en solution aqueuse 1M à une température de 0 à 5°C sur la solution aqueuse à 15 à 25 % d'héparine injectable, à un pH de 4 à 7 et une température de 70 à 100°C au reflux.

**4.** Procédé pour préparer un sel d'héparine selon la revendication 1, dans lequel le procédé comprend l'élimination du contre-ion inorganique de l'héparine de bas poids moléculaire par traitement avec une résine échangeuse d'ions en phase acide et la neutralisation subséquente du polysaccharide acide élué avec la triéthanolamine base (99 %) en ajustant le pH de l'éluat entre 5,0 et 5,5 à une température de 15 à 25°C.

**5.** Procédé selon la revendication 2, 3 ou 4, dans lequel le contre-ion inorganique est choisi parmi le sodium, le potassium, le calcium, le magnésium et l'ammonium.

**6.** Procédé selon la revendication 4, dans lequel l'héparine de bas poids moléculaire est obtenue par décomposition thermolytique d'une solution aqueuse d'acide hypochloreux à une température de 0 à 5°C sur une solution aqueuse à une concentration de 15 à 25 % d'héparine injectable à pH de 4 à 7 et à une température de 70 à 100°C au reflux.

**7.** Procédé selon la revendication 2 ou 4, dans lequel l'héparine de bas poids moléculaire est obtenue par la décomposition radicalaire de peroxyde d'hydrogène catalysée par des ions ferreux sur l'héparine injectable, la concentration en ion ferreux étant de 10 à 100 ppm, la concentration de peroxyde d'hydrogène en rapport molaire $H_2O_2$/polysaccharide étant de 6 à 15, le pH étant de 4 à 7 et la température réactionnelle étant de 60 à 120°C.

**8.** Procédé pour éliminer l'hygroscopicité d'un sel d'héparine de bas poids moléculaire avec la triéthanolamine selon la revendication 1, dans lequel le procédé comprend les étapes consistant à dissoudre ledit sel dans de l'eau jusqu'à atteindre une concentration de 13 à 17 %, ajouter du mannitol ou du sorbitol en une quantité équivalant à 30 à 50 parties pour 60 parties dudit sel, dissoudre le tout en solution aqueuse et lyophiliser.

**9.** Composition pharmaceutique pour l'usage local en thérapie antithrombotique, qui comprend le sel d'héparine de bas poids moléculaire avec la triéthanolamine selon la revendication 1, dans laquelle ledit sel est présent en des quantités pharmaceutiquement actives de 100 à 2000 unités internationales anti $X_A$/g de composition pour l'usage local diluée dans un excipient approprié.

**10.** Composition pharmaceutique selon la revendication 9, dans laquelle l'excipient est choisi parmi des gels aqueux de carbopolymères, des crèmes ou des émulsions du type huile dans l'eau, ou des pommades à base aqueuse ou hydro-alcoolique.

**11.** Utilisation de sel d'héparine de bas poids moléculaire avec la triéthanolamine pour la fabrication d'un médicament qui est un agent thérapeutique antithrombotique pour l'administration locale, dans laquelle il est utilisé pour le traitement, le soulagement ou la prévention de veines variqueuses, d'hémorroïdes, d'hématomes traumatiques ou post-chirurgicaux ou de phlébite.

**12.** Utilisation selon la revendication 11, dans laquelle l'hématome post-chirurgical provient d'une chirurgie plastique ou d'une restauration faciale.

**13.** Utilisation selon la revendication 11, **caractérisée en ce que** la phlébite est provoquée par la perfusion intraveineuse d'agents chimiothérapeutiques.

FIG. 1

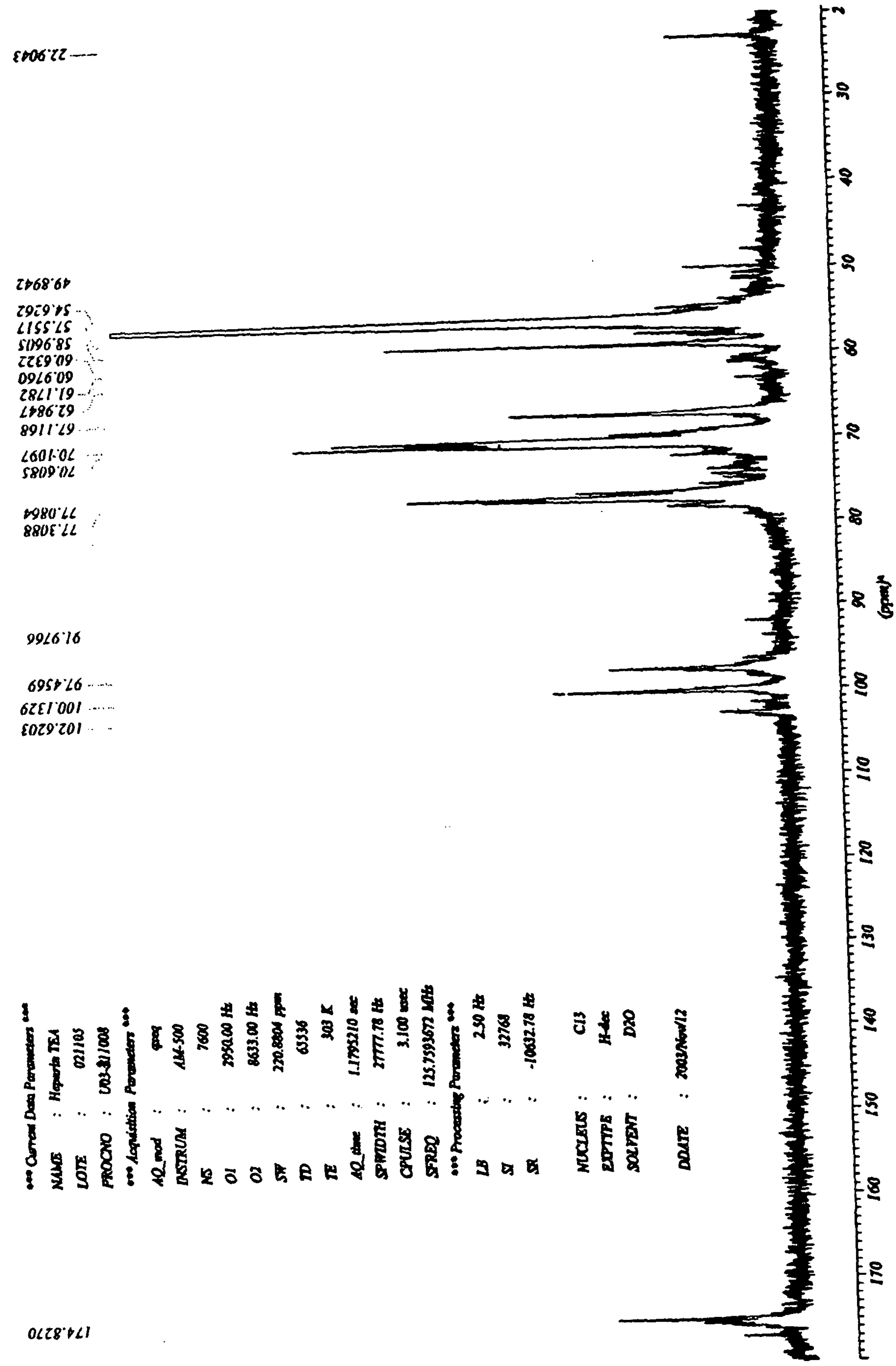

FIG. 2

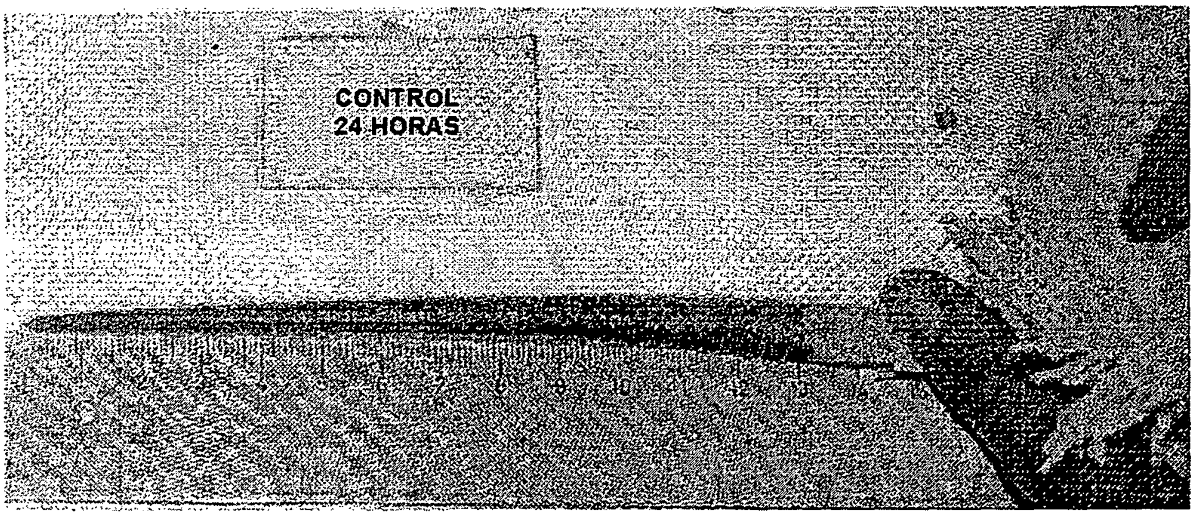

FIG. 3

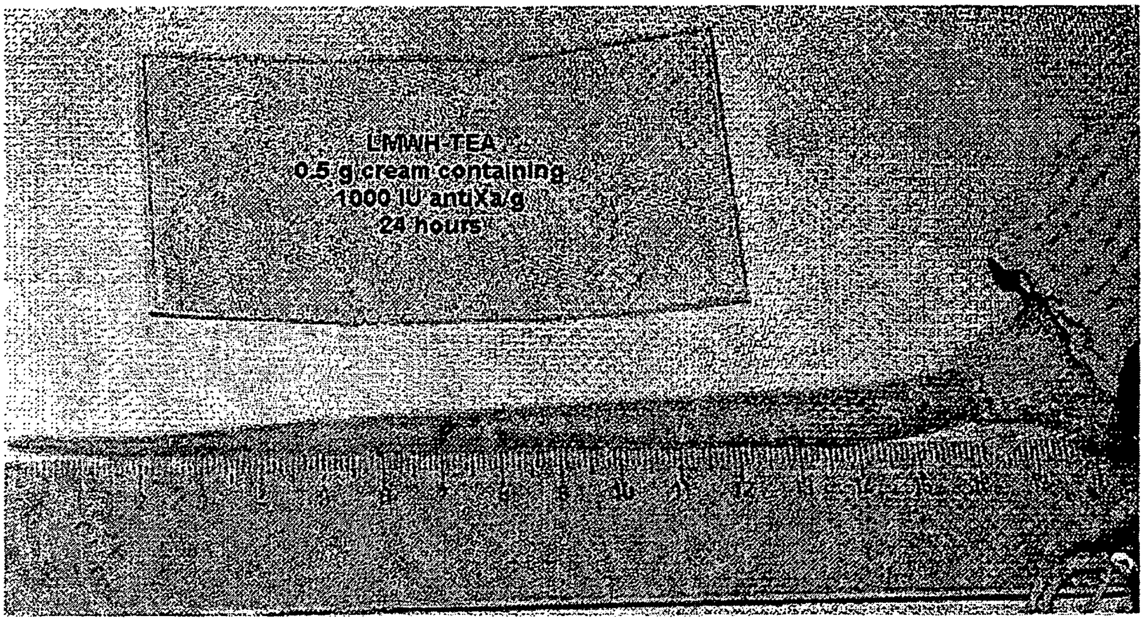
LMWH-TEA
0.5 g cream containing
1000 IU antiXa/g
24 hours

FIG. 4

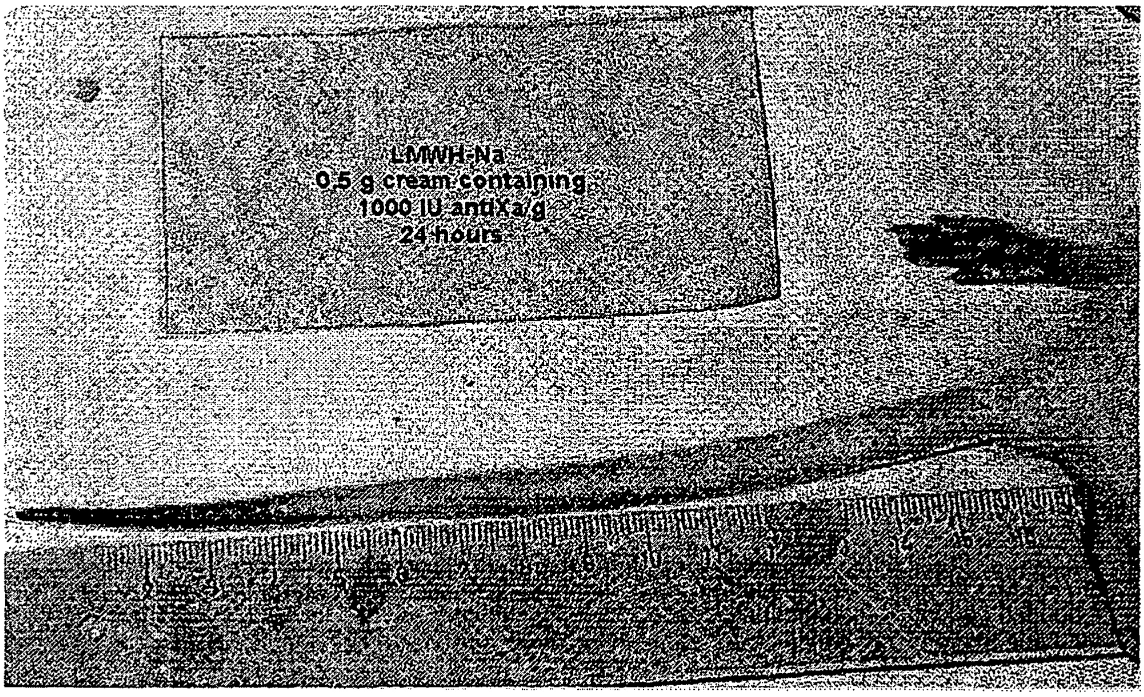
LMWH-Na
0.5 g cream containing
1000 IU antiXa/g
24 hours

FIG. 5

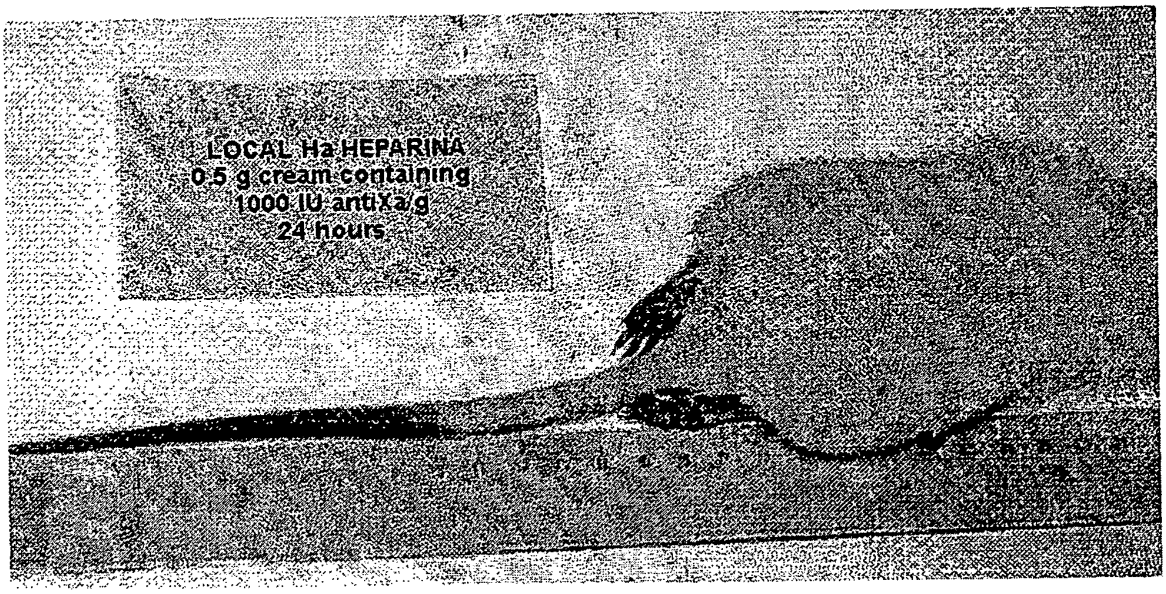
LOCAL Ha HEPARINA
0.5 g cream containing
1000 IU antiXa/g
24 hours

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0268885 A
- AR 243540 A
- ES 2007373 A
- IT 1224260 A
- BE 8700861 A
- EP 268885 A
- US 4977250 A


### Non-patent literature cited in the description


- **CAPPELLETTI, R et al.** *Anal. Biochem.,* 1979, vol. 99, 311-315
- Methods of Structural Analysis. **CASU, B.** Heparin, Chemical and Biological Properties, Clinical Applications. 1989, 25-49
- **BECKMEIER et al.** *Agents and Actions,* 1985, vol. 16, 446